Europäisches Patentamt

(19) European Patent Office

Office européen des brevets

(11) EP 0 308 536 B2

(12) NEW EUROPEAN PATENT SPECIFICATION

(45) Date of publication and mention
of the opposition decision:
**23.06.1999 Bulletin 1999/25**

(45) Mention of the grant of the patent:
**19.01.1994 Bulletin 1994/03**

(21) Application number: **87113984.6**

(22) Date of filing: **24.09.1987**

(51) Int. Cl.⁶: **A61N 1/368**

(54) **Pacemaker having programmable configuration**

Herzschrittmacher mit programmierbarer Gestaltung

Stimulateur cardiaque à configuration programmable

(84) Designated Contracting States:
**DE FR GB IT NL SE**

(43) Date of publication of application:
**29.03.1989 Bulletin 1989/13**

(73) Proprietor: **PACESETTER, INC.**
**Sylmar, CA 91392-9221 (US)**

(72) Inventor: **Silvian, Sergiu**
**Pasadena, CA 91107 (US)**

(74) Representative:
**Rees, David Christopher et al**
**Kilburn & Strode**
**20 Red Lion Street**
**London WC1R 4PJ (GB)**

(56) References cited:
**EP-A- 0 114 679          DE-A- 2 342 030**
**DE-A- 3 237 198          US-A- 4 379 459**
**US-A- 4 498 478          US-A- 4 549 548**

## Description

The present invention relates to implantable pacemakers capable of pacing and sensing in at least one chamber of the heart. More particularly, the present invention, relates to a programmable dual chamber pacemaker wherein the basic configuration of the pacemaker, e.g., unipolar or bipolar, can be changed, including the grounding configuration and ground potentials used within the pacemaker, and more exactly the present invention relates to an implantable pacemaker comprising a first electrode terminal, a first return electrode terminal, a second return electrode terminal, a voltage source having a positive voltage terminal and a negative voltage terminal, said positive voltage terminal defining a ground reference potential, stimulation potential generating means for generating a stimulation potential, first switch means for connecting said first electrode terminal via a coupling capacitor to said stimulation potential during a stimulating time period, second and third switching means for selectively connecting said first return electrode terminal or said second return electrode terminal to said ground reference potential during said stimulating time period, sensing means having a first and a second input terminal for sensing electrical signals therebetween, sensing configuration means for electrically connecting said sensing means to selected two of said electrode terminals. Such a pacemaker is previously known through EP-A-114 679.

Generally, a heart stimulator, commonly known as a "pacemaker" or "pacer" uses one or two flexible leads having one end connected to the pacer and the other end connected to electrodes placed in close proximity to the heart. These leads are used to stimulate or pace the heart. Also, these leads are used to sense the heart activity by picking up electrical signals from the heart.

In order to properly pace or sense, the pacer has to be able to deliver a stimulating pulse to the heart or sense an electrical signal from the heart, and this requires that there be an electrical return path. If, within a given heart chamber, a unipolar lead is used -- containing a single conductor -- the return path is the conductive body tissue and fluids. The return path is connected to the pacer by connecting the pacer electrical common or ground to the pacer metal enclosure, typically referred to as the pacer "case." The case, in turn, makes contact with the body tissue and/or fluids.

An alternative solution to using a unipolar lead in a given heart chamber is to use a double lead/electrode in the heart chamber, known as a bipolar lead. In a bipolar lead, a second conductor is spiraled over and insulated from a first conductor along the length of the lead. At the distal end of the lead, one of the conductors is connected to a first electrode, referred to as the "tip" electrode, and the second conductor is connected to a second electrode, referred to as a "ring" electrode. The ring electrode is generally situated 10 to 20 mm from the tip electrode. The tip electrode is typically placed in contact with heart tissue, while the ring electrode is in electrical contact with the blood. Because both body tissue and fluids are conductive, the ring electrode of a bipolar lead, in contact with the body fluids, serves as an electrical return for both pacing and sensing.

As indicated, pacing or sensing using the pacer case or enclosure as part of the electrical return path is known as unipolar pacing or sensing. Pacing or sensing using the lead ring electrode and associated lead conductor as the electrical return path is known as bipolar pacing or sensing.

There are numerous factors to consider when deciding whether unipolar or bipolar pacing and/or sensing should be used. Bipolar pacing has, in general, the advantage of requiring less energy than unipolar pacing. Further, bipolar sensing is less prone to crosstalk than is unipolar sensing. (Crosstalk, for purposes of this application, refers to a pacer mistakenly sensing a heart activity in one heart chamber immediately after the other chamber is paced.) Bipolar sensing reduces crosstalk resulting from unipolar or bipolar pacing in the opposite chamber.

Unipolar pacing and sensing offers the advantage, in general, of simpler circuitry within the pacemaker and a smaller diameter lead. Further, some doctors prefer unipolar over bipolar pacing and/or sensing as a function of other implantation and heart conditions. Usually, the pacer has a factory-set configuration, but in the last five years some programmable configuration pacers have appeared.

In addition to the conventional unipolar and bipolar sensing configurations, a new sensing configuration has the potential of reducing even more the likelihood of cross-talk. This new configuration utilizes unipolar pacing in both channels, and senses between the ring electrode and the case. See US-A-4 686 988. Not only is the crosstalk smaller with this new configuration, but one can readily determine capture just immediately after pacing (capture is defined as the heart contracting as a result of a pacer delivered stimulus).

As the number of configuration options and their combinations increases, especially with respect to dual chamber pacers (those designed to pace and/or sense in both chambers of the heart), it is clear that pacing and sensing programmability is very important. However, because a pacer is a low voltage, low power-consumption device, the implementation of the switching circuitry needed to realize the different pacing and sensing configurations is very difficult. To illustrate, in order to have a very low power-consumption device, pacers use integrated circuits with CMOS digital circuits and MOS analog switches and amplifiers. Further, low voltage, power and polarity requirements dictate the use of a P-well CMOS process. (A pacer is typically a positive ground system inasmuch as negative pacing pulses must be generated.) A difficulty with this CMOS process, and the resulting CMOS currents, is that no input, output, or any internal transistor drain or source can go above $V_{DD}$ or below $V_{SS}$, where $V_{DD}$ is the positive supply voltage and $V_{SS}$ is the negative supply voltage. (For a single battery configuration, $V_{DD}$ is thus usually obtained from the positive battery terminal, and $V_{SS}$ from the negative battery terminal.) Because the battery of a pacemaker is typically a single 2.8 volt (V) lithium cell, whose voltage may decrease over its life to as low as 2.0 V, this limitation makes it extremely difficult to design pacemaker circuits that will work properly in all output (pacing) and sensing configurations.

In a typical design, the pacer electrical common, or ground reference, is connected to the positive terminal of the battery. In turn, this ground reference is connected to the CMOS IC substrate. The negative terminal of the battery, which for a typical design is -2.8 V, thus provides the $V_{SS}$ supply voltage for the pacer circuits. As pacing magnitudes greater than 2.8 V are often required, a charge pump is used in conjunction with a storage capacitor for each channel of the pacemaker in order to produce these higher magnitude voltages. Also such charge pumps, or equivalent, can be used to produce some other higher magnitude voltages needed for circuits which have node voltages of greater magnitude than -2.8 V.

However, even though charge pump circuits can be used to produce needed voltages of greater magnitude than is available from the battery, a major problem still exists for nodes having voltages going above $V_{DD}$ or ground. An example will illustrate how such voltages occur. A pacer delivers a stimulating current pulse by switchably connecting the electrode tip, through a coupling capacitor, to the negative terminal of a storage capacitor, the positive terminal of this capacitor being grounded. The voltage stored on this storage capacitor has previously been "pumped up" to the desired magnitude by a charge pump circuit. A coupling capacitor is required to prevent DC current from flowing through the tip electrode-body interface. The return path for the pacing pulse is provided by grounding the case or ring electrode for unipolar or bipolar pacing, respectively. After delivering the pulse, the coupling capacitor remains charged with a positive charge on its tip electrode side (distal side). The pacer side of the coupling capacitor (proximal side) would likewise have a charge remaining thereon, but this charge is removed by connecting it through a discharging resistor (or switch) to ground. If, after pacing, it is desired to sense bipolarly between tip and ring, switching means must be used to connect the two inputs of a differential amplifier to the tip and ring electrodes. However, the tip potential remains above ground and the ring potential, situated in close proximity to the tip, has a potential somewhere between ground potential and the tip potential, but definitely above ground. As mentioned, no solid state switch of the type employed in pacer circuits (e.g., CMOS switch) can go above ground. Hence, a problem exists of how to switchably connect the positive (above ground) potentials of the tip and ring electrodes to the sense amplifier. One possible solution to this problem is to connect the sensing amplifier to the proximal (negative) side of the coupling capacitor, which proximal side will have a potential below ground due to the discharge current through the discharging resistor or switch. This approach has the drawback, however, of applying the capacitor's discharging voltage slope to the sensing amplifier. Further, the ring electrode would have to be connected through an additionnal coupling capacitor in order to eliminate its voltage potential above $V_{DD}$ (ground). Requiring an additional discrete component, such as a capacitor, is very undesirable.

A further possible solution to this switching problem would be to have the system ground different from $V_{DD}$, e.g., midway between $V_{DD}$ and $V_{SS}$. However, doing so would require a midway voltage source, to produce the midway ground potential, that is buffered by a low output impedance buffer to sustain the high current demands of pacing. Alternatively, the system ground could be connected to -2.8 V, the negative battery potential. However, doing so would require at least one more stage to the charge pump in order to produce the negative voltages required for pacing. The addition of such additional circuitry is undesirable because it would increase the bulk and power consumption of the pacer, as well as decrease its reliability.

SUMMARY OF THE INVENTION

The above and other problems are solved according to the invention with a pacemaker of the kind defined in the introduction which has the characterizing features of claim 1, thus by configuring or operating an otherwise conventional pacer by connecting the pacer case, or ground, to $V_{DD}$, the positive battery potential, during pacing (when the current demands are high) and by connecting the pacer case or ground to between -0.2 and -2.0 volts during sensing (when the current demands are low). Further, in one embodiment, a fast discharge circuit is employed in order to rapidly discharge the proximal side of a coupling capacitor through which the pacing pulse is delivered to a tip electrode. This fast discharge circuit switchably connects the proximal side of the coupling capacitor to both: (1) the case terminal or ring terminal (unipolar or bipolar, respectively), and (2) the negative battery potential for a prescribed time period subsequent to the delivery of the pacing pulse. During this fast discharge time period, the pacer case is disconnected from the positive battery potential. In still a further embodiment, this fast discharge time period is followed by a slow discharge period wherein the proximal side of the coupling capacitor is connected to the case terminal or ring terminal (unipolar or bipolar, respectively) through a discharge resistor.

Advantageously, all of the switching and resulting connections used to achieve the above configurations are realized using low power solid state switching devices, such as CMOS devices, that are controlled by appropriate state and timing signals. These state and timing signals, for the most part, are the same signals that are generated and used in a conventional programmable pacemaker. Such signals are digital signals, and as such, can be readily stored in the memory circuits of the pacemaker, or easily generated from control signals stored in such memory circuits, and recalled or generated as needed. Further, such signals can be easily altered or changed, using known telemetry and programming techniques, in order to allow the configuration of the pacer to be set to a desired configuration.

A feature of the present invention provides a pacemaker that can pace and/or sense in unipolar or bipolar modes of operation wherein the pacer ground is switched from the positive battery potential during pacing to a slightly more negative potential, in this case, between -0.2 and -2.0 volts, e.g., from -0.2 to -1.0 volts, during sensing. A still further feature of such a pacemaker, in accordance with one embodiment thereof, provides a fast discharge phase while in the pacing mode of operation. During this fast discharge phase the proximal side of the coupling capacitor (through which the pacing pulse is delivered to the tip electrode) is switchably connected to both: (1) the case terminal (unipolar operation) or the ring terminal (bipolar operation), and (2) the negative battery potential for a short time period, thereby causing the coupling capacitor to rapidly discharge. This fast discharge phase may be followed by a slow discharge phase. During this slow discharge phase the proximal side of this coupling capacitor is switchably connected to either the case (unipolar) or ring (bipolar) terminals through a resistor.

## BRIEF DESCRIPTION OF THE DRAWINGS

The above and other features and advantages of the present invention will be more apparent from the following more particular description thereof, presented in conjunction with the following drawings, wherein:

Fig. 1 is a simplified schematic diagram of the packing and sensing circuits of the present invention;
Fig. 2 is a timing diagram illustrating the operation of the pacing portion of the circuit of Fig. 1;
Fig. 2A is an equivalent circuit diagram of the circuit of Fig. 1 during the fast discharge time period;
Fig. 3 is a schematic diagram of the pacing or output portion of the present invention;
Fig. 4 is a schematic diagram of the sensing portion of the present invention; and
Fig. 5 is a table that defines the sensing configuration realized by the circuit of Fig. 4 as a function of the switches that are closed.

## DETAILED DESCRIPTION OF THE INVENTION

The following description is of the best presently contemplated mode of carrying out the invention. This description is not to be taken in a limiting sense but is made for the purpose of describing the general principles of the invention. The scope of the invention should be determined with reference to the appended claims.

In the description that follows, when reference is made to the elements or parts of the invention shown in the drawings, like numerals will be used to refer to like parts throughout.

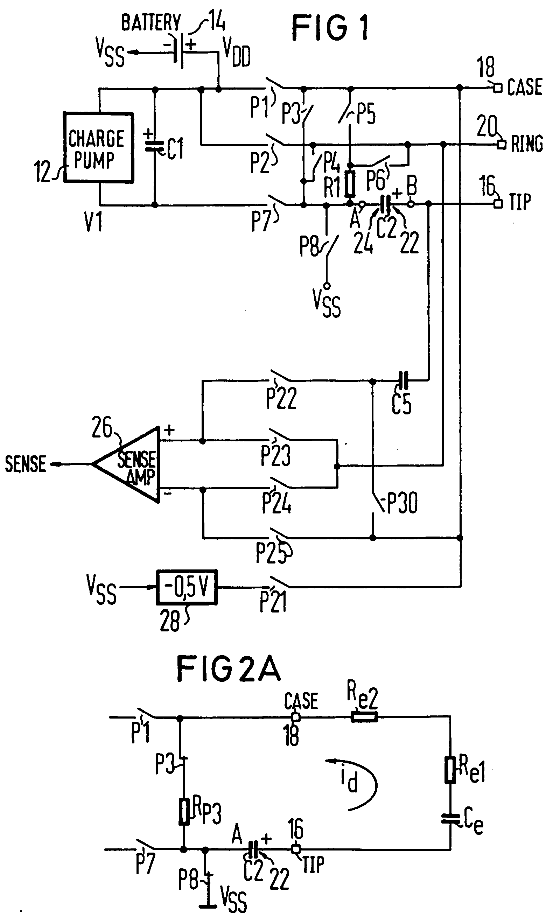

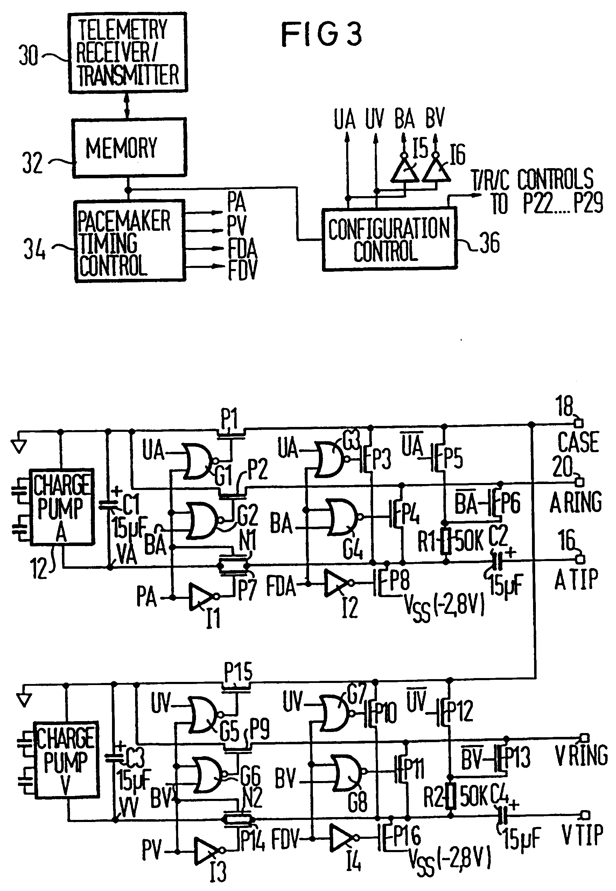

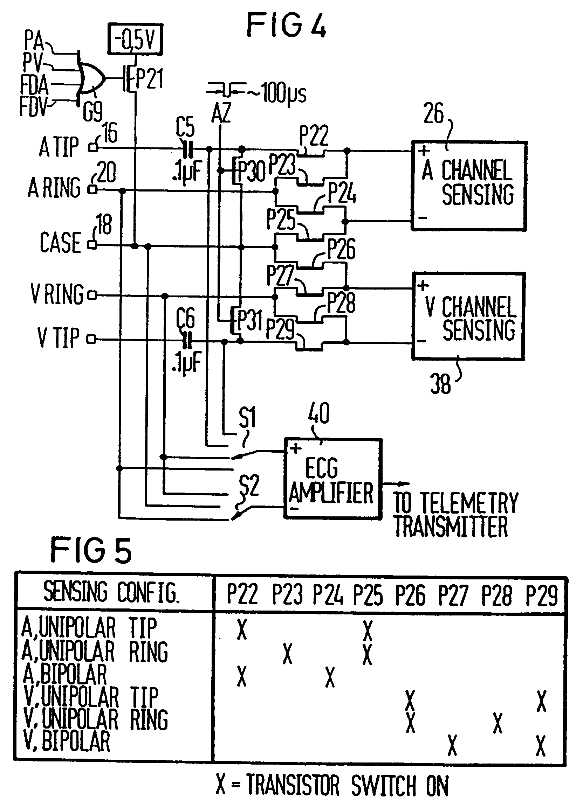

Referring first to Fig. 1, a simplified schematic diagram is shown of the pacing and sensing circuits of the present invention as used with one chamber of the heart. This figure will be used to teach the operating principles of the invention. A more detailed schematic diagram of the circuits of the invention can be found in Figs. 3 and 4.

In Fig. 1, the basic pacing circuit includes a charge pump circuit 12 and a storage capacitor C1. The charge pump circuit 12 takes the available battery potential $V_{SS}$ from battery 14, and pumps it up to a desired level in a conventional manner. During this process, one side of the charge pump 12 and the positive side of capacitor C1 are connected to ground potential, or $V_{DD}$. The stimulation pulse of desired magnitude is then delivered to a tip electrode terminal 16 by closing switch P7. The tip electrode terminal 16 is, in turn, connected to the tip electrode of a conventional pacing lead, not shown, thereby enabling the pacing pulse to be delivered to the heart. The return path for the stimulating pulse is provided either through the case terminal 18, for unipolar pacing, or the ring electrode terminal 20, for bipolar pacing. If unipolar pacing is selected, switch P1 is closed. If bipolar pacing is selected, switch P2 is closed.

Regardless of whether unipolar or bipolar pacing is selected, the pacing pulse must be delivered to the tip electrode terminal 16 through coupling capacitor C2. Capacitor C2 is needed in order to block DC currents from flowing from the storage capacitor C1 to the tip electrode terminal 16 and through the body tissue. It is noted that, as configured in Fig. 1, the pulse that is generated by closing switches P7 and P1 or P2 is a negative pulse relative to the pacer ground potential, $V_{DD}$. Generating a negative stimulation pulse in this or equivalent fashion is a requirement dictated by the physiology of the body and heart of the patient using the pacemaker. Hence, the pacemaker, during the pacing mode of operation, must essentially operate as a positive ground system if such a negative stimulation pulse is to be efficiently generated using a single battery 14. Further, because a negative stimulation or pacing pulse is generated, and because the body tissue on the distal side 22 of capacitor C2 is essentially at a zero potential, capacitor C2 is charged such that side 22 thereof retains a positive charge after the pacing pulse has passed therethrough.

In order to remove any residual charges from the proximal side 24 of capacitor C2 before the delivery of the next pacing pulse, and thereby prevent any such charges from adversely affecting the magnitude of the pacing pulse that is delivered, switch P3 (unipolar operation) or switch P4 (bipolar operation) creates a discharge path from the proximal side 24 of capacitor C2 to either the case terminal 18 (unipolar operation) or ring terminal 20 (bipolar operation) for a short period of time termed the "fast discharge" period. This fast discharge period immediately follows the delivery of the pacing pulse to the tip electrode. During this fast discharge period, switch P8 further connects side 24 of capacitor C2 to the negative battery potential $V_{SS}$. After the fast discharge period, in order to ensure that essentially all the charge is removed from side 24 of capacitor C2, a slow discharge path is provided through resistor R1 and switch P5 (unipolar operation) or switch P6 (bipolar operation). Hence, by the time the next pacing pulse needs to be generated, essentially all of the charge will have been removed from capacitor C2, thereby allowing a pacing pulse of known magnitude to be delivered to the tip electrode terminal 16.

Still referring to Fig. 1, sensing is realized by selectively connecting the two inputs of a conventional sense amplifier 26 to desired combination pairs of the tip electrode terminal 16, the ring electrode terminal 20, or the case electrode terminal 18. If conventional unipolar sensing is desired, the tip electrode 16 is connected to the positive terminal of sense amplifier 26 by closing switch P22; and the negative terminal of sense amplifier 26 is connected to the case terminal 18 by closing switch P25. If bipolar sensing is desired, the positive terminal of sense amplifier 26 is connected through switch P22 to the tip electrode 16, as with unipolar pacing, but the negative terminal of sense amplifier 26 is connected to the ring electrode through switch P24. If the new sensing mode is desired, then the positive terminal of sense amplifier 26 is connected to the ring electrode 20 by closing switch P23, and the case electrode 18 is connected to the negative terminal of sense amplifier 26 through switch P25. It is noted that all switches remain open unless specifically closed, thereby preventing two signals from being applied to the same sense amplifier terminal at the same time. It is also noted that coupling capacitor C5 is inserted in series with switch P22. This coupling capacitor C5 prevents the DC voltage component of any positive voltages that may be present on the tip electrode 16 from adversely affecting the operation of switch P22 or the other CMOS circuits and devices employed. There is no similar coupling capacitor employed for the ring electrode.

Most significantly, the sensing circuits shown in Fig. 1 include switch P21 that connects a different reference potential, other than $V_{DD}$, to the case or ground terminal 18 during the sensing operation. In the preferred embodiment, this different reference potential is -0.5 volts, and is generated by voltage reference generator circuit 28. Because the current demands during the sensing mode of operation are light, the circuitry of the reference generator 28 can be very simple, such as a voltage divider network comprised of a resistor and a diode. Using this additional reference voltage as the ground reference during sensing in this fashion assures that any slight positive voltages that may be present on the tip or ring electrodes will not exceed the $V_{DD}$ potential applied to the substrate of the solid state switches that are used.

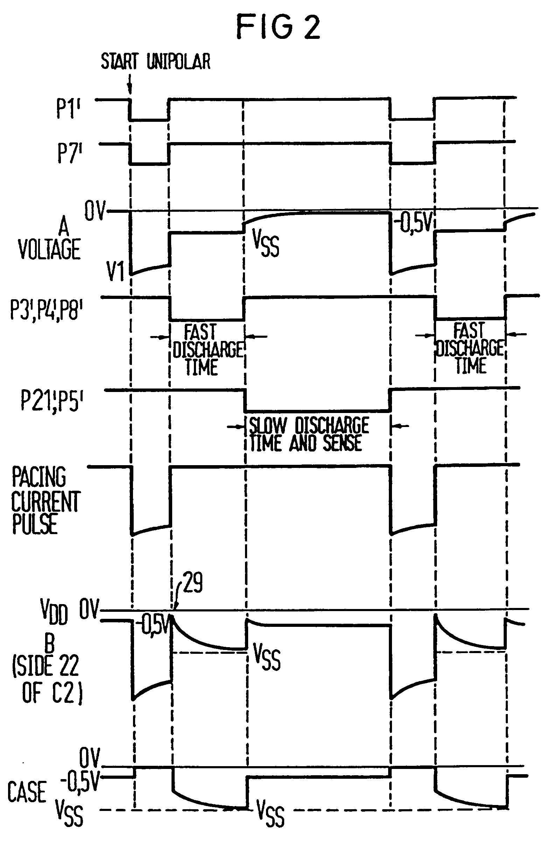

Fig. 2 depicts a simplified timing diagram illustrating the operation of the circuit of Fig. 1. Fig. 2 assumes a unipolar mode of operation has been selected, beginning at the point indicated on the left of the figure. Fig. 2 also assumes that the selected amplitude of the pulse to be delivered is roughly three times the available battery voltage, or $3V_{SS}$. The logic signals P1', P5', P7', and P8' shown in Fig. 2 are used to respectively control the P-MOS switches P1, P5, P7 and P8 of Fig. 1. A low level signal turns the switch on (closes the switch), while a high level signal turns the switch off (opens the switch).

As can be seen from Fig. 2, the closure of switches P7 and P1 causes the voltage V1, present on capacitor C1, to be applied across terminals 16 and 18. In turn, this causes a pacing current pulse to flow assuming that the tip electrode 16 and the case electrode 18 are in contact with conductive body tissue (or some other load). The waveform identified as "A" in Fig. 2 corresponds to the voltage waveform appearing at point "A" in Fig. 1, which point or node corresponds to the proximal side 24 of capacitor C2. This waveform "A" initially drops to voltage V1 when switch P7 is closed. As soon as P1 and P7 open, switch P3 and switch P8 close to begin the fast discharge time. During this fast discharge time, switch P1 also opens, thereby disconnecting the case electrode 18 from $V_{DD}$. Hence, during this fast discharge time, most of the charge remaining on side 24 of capacitor C2 is discharged through switch P3. Hence, as shown in Fig. 2, at the conclusion of the pacing pulse (when P7 and P1 open and P3 and P8 close), the fast discharge time begins and the voltage waveform "A" rises to $V_{SS}$. After the fast discharge time has elapsed, a slow discharge time begins and the pacemaker must be ready to sense signals appearing at the tip and/or ring electrode. Hence, switch P21 closes, thereby connecting the case electrode 18 to -0.5V, and switch P5 also closes, thereby providing a slow discharge path through resistor R1 and switch P5. This additional slow discharge time ensures that essentially all remaining charge on capacitor C2 is removed before the next pacing pulse is generated. Hence, waveform "A" slowly rises to the -0.5V level during the slow discharge time. Before the next pacing pulse is generated, capacitor C2 has essentially discharged.

Also shown in Fig. 2 is the voltage waveform appearing at the distal side 22 of coupling capacitor C2. This voltage is identified as waveform "B." Initially, this voltage is at -0.5 volts, the reference level that is applied to the case during sensing. During the pacing pulse, this voltage goes negative as it follows the negative voltage applied to the proximal side 24 of C2. After the pacing pulse, this voltage attempts to go positive, but by connecting the proximal side 22 of C2 to $V_{SS}$ at this time, this voltage does not go above $V_{DD}$ its most positive point 29, waveform "B" advantageously remains below $V_{DD}$. During the remaining portion of the fast discharge time, waveform "B" approaches $V_{SS}$, the voltage level applied to the other (proximal) side of capacitor C2. At the end of the fast discharge time, the sense period begins during which the case is connected to -0.5 V through P21, and during which time the proximal side of C2 is connected through resistor R1 and P5 to the case. Hence, the distal side of C2 also approaches this same voltage level (-0.5 V).

Further shown in Fig. 2 is the case voltage. As can be seen, and as explained previously, this case voltage is connected to $V_{DD}$ (O V) during the time the pacing pulse is delivered by the closure of switch P1. During the fast discharge time, switch P1 is opened and switches P3 and P8 are closed, disconnecting the case from $V_{DD}$ and connecting the case to $V_{SS}$. However, because of the internal resistance associated with switches P3 and P8, the case voltage does not immediately go to $V_{SS}$, but rather approaches $V_{SS}$. After the fast discharge period, the slow discharge period or sense period begins, during which the case is connected to -0.5 V through switch P21.

It is noted that during the fast discharge time period, the equivalent circuit for the pacemaker and lead is as shown in Fig. 2A. In Fig. 2A, resistance $R_{P3}$ is the resistance of switch P3 when closed (approximately 100 ohms); capacitance $C_e$ is the tip equivalent capacitance; resistance $R_{e1}$ is the tip equivalent resistance; and resistance $R_{e2}$ is the equivalent case, body return resistance. Typically, for a properly positioned tip electrode, $R_{e1}$ plus $R_{e2}$ will be equal to around 500 ohms. During fast discharge, the case terminal 18 is not directly connected to any pacer voltage source. Hence, case 18 assumes a voltage determined by the voltage on the distal side 22 of capacitor C2 and by the voltage divider comprising $R_{p3}$, $R_{e1}$ and $R_{e2}$ -(unipolar operation) or $R_{p4}$ (resistance of switch P4), $R_{e1}$, and $R_{e2}$ (bipolar operation). Mathematically, this case voltage can be expressed as

$$\text{CASE VOLTAGE} = V_{SS} + (\text{C2 voltage})\ \frac{R_{p3}}{R_{p3} + (R_{e1} + R_{e2})}$$

At the end of the fast discharge period, it is thus seen that the case voltage will approach $V_{SS}$ (because the C2 voltage approaches zero).

Referring next to Fig. 3, a more detailed schematic diagram of the pacing portion of the present invention is shown. Many of the elements or parts shown in Fig. 3 correspond to the same elements shown in Fig. 1. Hence, the same numerals or letters are used to identify such elements.

The pacemaker of the present invention includes telemetry receiving and transmitting circuitry 30. Such circuitry may be of conventional design. It is used to provide two-way communication with the pacemaker once it is implanted in a patient. Such two-way communication not only allows the pacemaker parameters to be programmed after pacemaker implant, but also allows signals sensed by the implanted pacemaker, or the operating status of the pacemaker, to be telemetered out of the implanted pacemaker to an external receiver.

Further included within the pacemaker is some sort of memory device or element 32. The memory 32 allows the controlling parameters of the pacemaker to be stored so they can provide the needed control for the pacemaker as required. Further, the memory 32 provides a convenient means for realizing any altering or reprogramming of the pacemaker configuration or operating modes. All that needs be done to make a programming change is to telemeter new controlling data to the appropriate address in the memory 32.

For purposes of the present invention, the operating circuits of the pacemaker include timing control logic 34 and configuration control logic 36. The pacemaker timing control logic 34 generates the signals that control when a pulse is to be provided to the atrium and the ventricle, and when the fast discharge time is to be present for the atrium and the ventricle. These signals are identified as: Pulse Atrium (PA); Pulse Ventricle (PV); Fast Discharge Atrium (FDA); and Fast Discharge Ventricle (FDV). The configuration control logic generates the signals that control whether the pacemaker is to operate in a bipolar or unipolar mode. These signals are identified as: Unipolar Atrium (UA); Unipolar Ventricle (UV); Bipolar Atrium (BP); and Bipolar Ventricle (BP). Further configuration control signals control whether unipolar operation during sensing is to be tip-to-case, ring-to-case, or bipolar. These signals are commonly identified as Tip/Ring/Case (T/R/C).

The pacing circuit of Fig. 3 includes two channels, one for pacing the atrium and one for pacing the ventricle. Only one case electrode 18 is shown because the case is common to both channels. The discussion that follows is directed to the atrial channel, but applies equally well to the ventricular channel inasmuch as the circuits for both channels, for purposes of the present invention, function the same.

The switches shown in Fig. 3 are realized with MOS switching devices, the gate terminal of which is controlled from logic gates. For example, switch P1 of Fig. 3 is a switch which is normally open unless a low level signal appears at the output of logic gate G1. As gate G1 is a two-input NAND gate having one input connected to the UA signal and the other connected to the PA signal, it is seen that switch P1 will close only in the presence of a UA and PA signal. Similarly, it is seen that gate G2 will close only in the presence of a BA and PA signal.

One switch per channel in Fig. 3 is realized using both a P-well and N-well MOS switch in parallel. This switch is configured as a transmission gate and is identified as the P7/N1 switch (corresponding to the P7 switch of Fig. 1). A

6

low level logic signal applied to the P7 side closes the P7 switch, while a high level logic signal applied to the N1 side closes the N1 switch. Inverter gate I1 assures that complementary signals are always applied to both sides of this switch, thereby assuring that the switch is either fully on or fully off. A dual switch is employed in this one position because the voltage on mode VA can be programmed to different values. For example, if the programmed value of VA is -0.5 V, a P-MOS transistor switch will work properly. However, if VA is close to - $V_{SS}$, an N-MOS transistor switch is required (which N-MOS transistor is turned on by a positive gate voltage, rather than the negative gate voltage used to turn on a P-MOS transistor). Hence, the P7/N1 switch configuration assures that this switch will close regardless of the programmed value of VA.

The operation of the pacing circuit of Fig. 3 parallels the operation described above of the circuit of Fig. 1, as explained using the timing diagram of Fig. 2. It is noted that during the fast discharge time period, capacitor C2 is connected to the negative battery potential $V_{SS}$. After the fast discharge period, resistor R1, connected through P5 or P6 (depending upon whether unpolar or bipolar pacing is selected) continues to discharge capacitor C2 at a lower rate.

As indicated, the ventricle channel circuits, comprising the circuits in the lower half of the schematic diagram of Fig. 3, function the same as the atrial channel circuits described above. It should be understood by those skilled in the art that the control signals UA, PA, FDA, UV, PV, and FDV are generated through appropriate level shifting circuits, and that inverter gates I1 through I6 and logic gates G1 through G8 have their positive supply terminals connected to $V_{DD}$, and their negative supply terminals connected to an appropriate supply voltage that is equal or lower than the negative peak of the delivered pulse. This negative voltage can be taken from the same charge pump circuits used to generate the V1 voltage for the pacing pulse, from the storage capacitors C1 or C3, or from any other negative source. This separate supply voltage for these particular gates is required to maintain the switches ON while their respective drain or source terminals go below the negative battery voltage, $V_{SS}$. Also, a larger (in absolute value) gate voltage applied to all of the P switches advantageously allows a reduction in the overall physical dimensions of the devices while maintaining the same ON resistance.

Referring next to Fig. 4 it is seen that there are two channel sensing amplifiers or circuits 26 and 38. Amplifier 26 senses and amplifies the millivolt-level signals generated in the atrium during each heart contraction. Similarly, amplifier 38 senses and amplifies the millivolt-level signals generated in the ventricle. Eight switching PMOS transistors, P22-P29, are controlled by sensing configuration stored data as set forth in Fig. 5. As indicated in Figs. 4 and 5, each channel can be programmed to sense unipolarly using the tip and case, unipolarly using the ring and case, or bipolarly using the tip and ring. It is noted that during the delivery of a pacing pulse, or during the fast discharge time period, of any channel, switches P22-P29 are switched OFF (open or very high impedance) to avoid saturating the sensing circuits 26 and 38. If no pulse is delivered, and if no fast discharge occurs, gate G9 controls switch P21 to turn it ON (closed or low resistance), connecting the case during sensing to -0.5 volts. This action allows some polarization as high as +0.5 volts on the ring electrodes without switches P23, P24, P27, or P28 going above $V_{DD}$. It is noted that while -0.5 volts is the preferred voltage for connecting the case or ground during sensing, it is only representative of one of a range of voltages that could be so used. For example, any voltage lying within the range of -0.2 to -2.0 volts could be used for this same purpose.

Still referring to Fig. 4, it is noted that two coupling capacitors, C5 and C6, prevent applying the voltages remaining on capacitors C2 and C4, after fast discharge, to P22 or P29. As noted in Fig. 2, waveform "A," it is possible that some voltage will remain on capacitors C2 or C4 at this time (after fast discharge), and this voltage could be misinterpreted by the sensing circuits 26 and 38 as cardiac activity. Accordingly, capacitors C5 and C6 are used to present such misinterpretation from occurring. To further ensure that any charge on capacitors C5 and C6 is removed, a short auto zero pulse of approximately 100 microseconds is used to discharge capacitors C5 and C6 through switches P30 and P31, respectively, just after the end of the fast discharge period. As seen in Fig. 4, switches P30 and P31 connect the proximal side of capacitors C5 and C6 to the case or ground terminal 18, which in turn is connected through switch P21 to -0.5 volts. This sensing configuration prevents any switch voltage from going above $V_{DD}$. During sensing, most of the switch voltages remain around -0.5 volts.

As is further shown in Fig. 4, one-pole switches S1 and S2 may also optionally be used to connect the input of an ECG amplifier 40 to the signals that are sensed through the tip and ring electrodes. Such signals comprise the intercardiac ECG signals that can then be processed and telemetered to an external receiver.

## Claims

1. An implantable pacemaker comprising a first electrode terminal (16), a first return electrode terminal (18), a second return electrode terminal (20), a voltage source (14) having a positive voltage terminal ($V_{DD}$) and a negative voltage terminal ($V_{SS}$), said positive voltage terminal defining a ground reference potential ($V_{DD}$), stimulation potential generating means (12, C1) for generating a stimulation potential (V1), first switch means (P7) for connecting said first electrode terminal (16) via a coupling capacitor (C2) to said stimulation potential (V1) during a stimulating time period, second and third switching means (P1, P2) for selectively connecting said first return electrode terminal (18) or said second return electrode terminal (20) to said ground reference potential (V$_{DD}$) during said stimulating time period, sensing means (26) having a first and a second input terminal for sensing electrical signals there-between, sensing configuration means (P21 - P25) for electrically connecting said sensing means (26) to selected two of said electrode terminals (16, 18, 20), characterised by means (28) for generating a second reference potential (-0.5 V) which has a value between -0.2 and -2.0 V with respect to the ground reference potential (V$_{DD}$) whereby the selected one of said return electrode terminals (18, 20) is connected to said second reference potential (-0.5 V) during a sensing time period.

2. The pacemaker of Claim 1, characterised in that said second reference potential (-0.5 V) has a value between -0.2 and -1.0 volt with respect to the ground reference potential (V$_{DD}$).

3. The pacemaker of Claim 1 or Claim 2, characterised by fast discharge means (P3, P8) for connecting the side (A) of said coupling capacitor (C2) which is connected to said first switch means (P7) to the selected one of said return electrode terminals (18, 20) and to a third reference potential (V$_{SS}$) during a fast discharge time period between said stimulating time period and said sensing time period, said third reference potential (V$_{SS}$) being equal to the potential at the negative voltage terminal (V$_{SS}$) of said voltage source (14).

4. The pacemaker of Claim 3, characterised by slow discharge means (R1, P5, P6) for connecting the side (A) of said coupling capacitor (C2) which is connected to said first switch means (P7) via a resistor (R1) to the selected one of said return electrode terminals (18, 20) during said sensing time period.

5. The pacemaker of one of the preceding Claims, characterised in that said first electrode terminal (16) is connected to a tip electrode, said first return electrode terminal (18) is connected to the case of said pacemaker and in that said second return electrode terminal (20) is connected to a ring electrode.

**Patentansprüche**

1. Implantierbarer Herzschrittmacher umfassend einen ersten Elektrodenanschluß (16), einen ersten Rückführungs-Elektrodenanschluß (18), einen zweiten Rückführungs-Elektrodenanschluß (20), eine Spannungsquelle (14), die einen Anschluß (V$_{DD}$) mit positiver Spannung und einen Anschluß (V$_{SS}$) mit negativer Spannung aufweist, wobei der Anschluß mit positiver Spannung ein Erd-Referenzpotential (V$_{DD}$) definiert, eine Stimuiationspotential-Erzeugungseinrichtung (12, C1) zur Erzeugung eines Stimulationspotentials (V1), eine erste Schaltereinrichtung (P7) zum Verbinden des ersten Elektrodenanschlusses (16) über einen Kopplungskondensator (C2) mit dem Stimulationspotential (V1) während einer Stimulationszeitperiode, zweite und dritte Schaltereinrichtungen (P1, P2) zum selektiven Verbinden des ersten Rückführungs-Elektrodenanschlusses (18) oder des zweiten Rückführungs-Elektrodensnschlusses (20) mit dem Erdreferenzpotentail (V$_{DD}$) während der Stimulationszeitperiode, eine Sensoreinrichtung (26), die einen ersten und einen zweiten Eingangsanschluß zum Abtasten von elektrischen Signalen zwischen diesen aufweist, eine Sensor-Konfigurierungseinrichtung (P21-P25), um die Sensoreinrichtung (26) mit zwei ausgewählten der Elektrodenanschlüsse (16, 18, 20) zu verbinden, **gekennzeichnet durch** eine Einrichtung (28) zur Erzeugung eines zweiten Referenzpotentials (-0.5 V), das einen Wert zwischen -0.2 und -2.0 V im Bezug auf das Erdreferenzpotential (V$_{DD}$) hat, wodurch der ausgewählte der Rückführungs-Elektrodenanschlüsse (18, 20) mit dem zweiten Bezugspotential (-0.5 V) während der Sensorzeitperiode verbunden wird.

2. Herzschrittmacher nach Anspruch 1, **dadurch gekennzeichnet, daß** das zweite Referenzpotential (-0.5 V) einen Wert zwischen -0.2 und -1.0 V im Bezug auf das Erd-Referenzpotential (V$_{DD}$) hat.

3. Herzschrittmacher nach Anspruch 1 oder 2, **gekennzeichnet durch** eine Schnellentladungseinrichtung (P3, P8) zum Verbinden der Seite (A) des Kopplungskondensators (P2), der mit der ersten Schalteinrichtung (P7) verbunden ist, mit einem ausgewählten der Rückführungs-Elektrodenanschlüsse (18, 20) mit einem dritten Referenzpotential (V$_{SS}$) während einer Schnellentladungszeitdauer zwischen der Stimulationszeitdauer und der Sensorzeitdauer, wobei das dritte Referenzpotential (V$_{SS}$) gleich dem Potential an dem negativen Spannungsanschluß (V$_{SS}$) der Spannungsquelle (14) ist.

4. Herzschrittmacher nach Anspruch 3, **gekennzeichnet durch** eine Einrichtung (R1, P5, P6) zur langsamen Entladung, um die Seite (A) des Kopplungskondensators (C2), der mit der ersten Schaltungseinrichtung (P7) verbunden ist, über einen Widerstand (R1) mit dem ausgewählten der Rückführungs-Elektrodenanschlüsse (18, 20) während der Sensorzeitperiode zu verbinden.

5. Herzschrittmacher nach einem der vorhergehenden Ansprüche, **dadurch gekennzeichnet, daß** der erste Elektrodenanschluß (16) mit einer Spitzenelektrode verbunden ist, daß der erste Rückführungselektrodenanschluß (18) mit dem Gehäuse des Herzschrittmachers verbunden ist und daß der zweite Rückführungs-Elektrodenanschluß (20) mit einer Ringelektrode verbunden ist.

## Revendications

1. Stimulateur cardiaque implantable comprenant une première borne d'électrode (16), une première borne d'électrode retour (18), une seconde borne d'électrode de retour (20), une source de tension (14) ayant une borne de tension positive ($V_{DD}$) et une borne de tension négative ($V_{SS}$), cette borne de tension positive définissant un potentiel de référence de masse ($V_{DD}$), des moyens de génération de potentiel de stimulation (12,C1), destinés à générer un potentiel de stimulation (V1), un premier élément de commutation (P7) destiné à connecter la première borne d'électrode (16) au potentiel de stimulation (V1) par l'intermédiaire d'un condensateur de couplage (C2), pendant une période de stimulation, des second et troisième éléments de commutation (P1, P2) pour connecter sélectivement la première borne d'électrode de retour (18) ou la seconde borne d'électrode de retour (20) au potentiel de référence de masse ($V_{DD}$) pendant la période de stimulation, des moyens de détection (26), ayant des première et seconde bornes d'entrée, pour détecter les signaux électriques présents entre elles, des moyens de définition de configuration de détection (P21-P25) destinés à connecter électriquement les moyens de détection (26) à deux bornes sélectionnées parmi les bornes d'électrodes (16,18,20), caractérisé par des moyens (28) qui sont destinés à générer un second potentiel de référence (-0,5 V), qui a une valeur entre (-0,2 et -2,0V) par rapport au potentiel de référence de masse ($V_{DD}$), grâce à quoi la borne sélectionnée parmi les bornes d'électrodes de retour (18,20) est connectée au second potentiel de référence (-0,5V) pendant une période de détection.

2. Stimulateur cardiaque selon la revendication 1, caractérisé en ce que le second potentiel de référence (-0,5 V) a une valeur entre -0,2 et -1,0 volts par rapport au potentiel de référence de masse ($V_{DD}$).

3. Stimulateur cardiaque selon la revendication 1 ou 2, caractérisé par des moyens de décharge rapide (P3, P8) pour connecter le côté (A) du condensateur de couplage (C) qui est connecté au premier élément de commutation (P7) à la borne sélectionnée parmi les bornes d'électrodes de retour (18, 20), et un troisième potentiel de référence ($V_{SS}$), pendant une période de décharge rapide entre la période de stimulation et la période de détection, le troisième potentiel de référence ($V_{SS}$) étant un égal au potentiel à la borne de tension négative ($V_{SS}$) de la source de tension (14).

4. Stimulateur cardiaque selon la revendication 3, caractérisé par des moyens de décharge lente (R1, P5, P6) qui sont destinés à connecter le côté (A) du condensateur de couplage (C2) qui est connecté au premier élément de commutation (P7), par l'intermédiaire d'une résistance (R1) à la borne sélectionnée parmi les bornes d'électrodes de retour (18, 20) pendant la période de détection.

5. Stimulateur cardiaque selon l'une des revendications précédentes, caractérisé en ce que la première borne d'électrode (16) est connectée à une électrode de pointe, la première borne d'électrode de retour (18) est connectée au boîtier du stimulateur cardiaque et en ce que la seconde borne de l'électrode de retour (20) est connectée à une électrode annulaire.

FIG 1

FIG 2A

# FIG 2

FIG 3

# FIG 4

# FIG 5

| SENSING CONFIG. | P22 | P23 | P24 | P25 | P26 | P27 | P28 | P29 |
|---|---|---|---|---|---|---|---|---|
| A, UNIPOLAR TIP | X | | | X | | | | |
| A, UNIPOLAR RING | | X | | X | | | | |
| A, BIPOLAR | X | | X | | | | | |
| V, UNIPOLAR TIP | | | | | X | | | X |
| V, UNIPOLAR RING | | | | | X | | X | |
| V, BIPOLAR | | | | | | X | | X |

X = TRANSISTOR SWITCH ON